(19) Europäisches Patentamt
European Patent Office
Office européen des brevets

(11) Publication number: 0 256 180 B1

# (12) EUROPEAN PATENT SPECIFICATION

(45) Date of publication of the patent specification: **27.12.89**

(51) Int. Cl.⁴: **C07D 215/14**, C07D 215/20, C07D 215/18, B41M 5/12, B41M 5/26, C09B 23/14

(21) Application number: **86306201.4**

(22) Date of filing: **11.08.86**

(54) **Chromogenic compounds for pressure- or heat-sensitive recording papers.**

(43) Date of publication of application: **24.02.88 Bulletin 88/8**

(45) Publication of the grant of the patent: **27.12.89 Bulletin 89/52**

(84) Designated Contracting States: **BE CH DE FR GB IT LI SE**

(56) References cited:
**DE-A- 3 219 239**
**GB-A- 2 136 823**
**US-A- 4 287 335**

(73) Proprietor: **YAMADA CHEMICAL CO., LTD., 1-1 Kamitoba-Kamichoshicho Minami-ku, Kyoto-shi Kyoto-fu(JP)**

(72) Inventor: **Fujisaki, Hideaki, 6-304 Jujo-Danchi 40 Kisshoin-Minami-Ochiaicho, Minami-ku Kyoto-shi Kyoto-fu(JP)**
Inventor: **Mizuno, Kozo, 19-35 Kohatakumakoji, Uji-shi Kyoto-fu(JP)**
Inventor: **Uda, Yoshinori, 103 Kamo-Haitsu 1-4 Kamoda Kamiueno-cho, Muko-shi Kyoto-fu(JP)**
Inventor: **Sueto Yukihiko, 175-10 Kuse-Minamigaito, Joyo-shi Kyoto-fu(JP)**
Inventor: **Tsunemitsu, Katsuhiko, 30-3 Daigo-Takahatacho Fushimi-ku, Kyoto-shi Kyoto-fu(JP)**

(74) Representative: **Woods, Geoffrey Corlett et al, J.A. KEMP & CO. 14 South Square Gray's Inn, London WC1R 5EU(GB)**

EP 0 256 180 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

ACTORUM AG

## Description

The present invention relates to a novel chromogenic compound, color developing composition containing the same suitable for recording paper, and pressure- or heat-sensitive recording paper coated with microcapsules containing the color developing composition.

Heretofore, a variety of yellow color forming agents have been proposed and they are broadly classified into the following two groups.

Group I: Those having a lactone ring in the molecule.

(i) Fluoran derivatives such as 3,6-dialkoxyfluoran (refer to JP-B 454 698 [1970] and 4 616 053 [1971]) and 3-N-alkylaminofluoran (refer to JP-B 4 622 650 [1971] and 484 051 [1973]).

(ii) Chromenopyrazole compounds (refer to JP-B 4 623 513 [1971]).

(iii) Aminophthalide compounds (refer to JP-A 54 111 528 [1979]).

(iv) Acyloxytetrachlorophthalide compounds (refer to JP-B 4 525 654 [19710]).

Group II: Those having no lactone ring in the molecule.

(i) Spiropyran derivatives (refer to JP-B 4 610 075 [1971] and 4 611 113 [1971]).

(ii) Styryl compounds (refer to JP-B 4 121 033 [1966] and 5 127 169 [1976]).

(iii) Pyridine derivatives (refer to JP-B 539 127 [1978]).

(iv) Monomethine compounds (refer to JP-B 5 223 406 [1977] and JP-B 495 929 [1974]).

(v) Benzopyran compounds (refer to JP-B 5 619 274 [1981]).

The compounds belonging to Group I which have a lactone ring in the molecule have an advantage of being less liable to spontaneous color formation in case of coating the solution thereof with a paper and being readily soluble in solvents. However, they also have a disadvantage of giving rise to a color image which is poor in color density and light-fastness.

On the other hand, the compounds belonging to Group II which have no lactone ring in the molecule have an advantage of producing a color image which is good in color density and light-fastness. However, they also have a disadvantage of being poor in solubility, being liable to spontaneous color formation in case of contact with paper, and being readily lost by sublimation.

Previously to the present invention, two of the present inventors as a part of inventors completed an invention relating to:

(I) a chromogenic compound of the formula

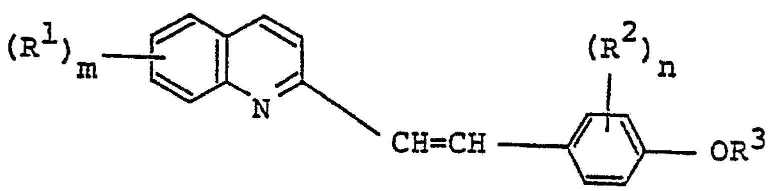

wherein $R^1$ and $R^2$ independently represent a hydrogen atom, lower alkyl group of l to 5 carbon atoms, lower alkoxy group of l to 5 carbon atoms, or halogen atom; $R^3$ represents a hydrogen atom, alkyl group of l to l2 carbon atoms, alkoxyalkyl group of l to l2 carbon atoms, halogenoalkyl group of l to l2 carbon atoms, phenyl group which may be substituted, or benzyl group which may be substituted; and m and n are an integer of l or 2; providing that $R^1$ and $R^2$ do not represent a hydrogen atom, methyl group, and methoxy group when $R^3$ represents a hydrogen atom or methyl group;

(2) a color forming composition for a recording material, comprising at least one chromogenic compound as defined above which is capable of developing a color upon contact with an electron acceptor; and

(3) pressure- or heat-sensitive recording paper comprising microcapsules containing at least one chromogenic compound as defined above, said chromogenic compound functioning as a color former and being dissolved in an organic solvent in said microcapsules (refer to GB-A 2 136 823).

the present inventors carried out studies for obtaining a new chromogenic compound which is easier to synthesize and more soluble than the ones disclosed in the previous publications mentioned above, and as a result, found compounds that are easy to synthesize, readily soluble, stable to light, and in addition, develop a color image of deep hue.

in a first aspect of the present invention, there is provided a chromogenic compound represented by the formula (I):

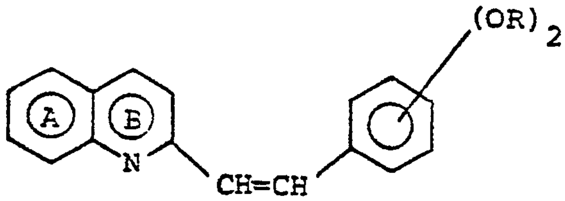

(I)

wherein R represents a $C_6$–$C_{12}$ alkyl group or benzyl group and the quinoline ring is unsubstituted or substituted in the 6-position by a $C_1$–$C_4$ alkoxy group or a $C_1$–$C_4$ alkyl group or a halogen atom.

In a second aspect of the present invention, there are provided microcapsules suitable for use in the preparation of a pressure- or heat-sensitive recording paper, in which microcapsules there is encapsulated a solution of at least one chromogenic compound of formula (I) in an organic solvent therefor.

In a third aspect of the present invention, there is provided a pressure- or heat-sensitive recording paper incorporating at least one chromogenic compound of formula (I) as color former.

In a fourth aspect of the present invention there is provided the use as a color former for pressure- or heat-sensitive recording papers of at least one chromogenic compound of formula (I).

Preferably R represents an alkyl group of 8 to 12 carbon atoms and/or one of the OR substituents is in the ortho-position with respect to the ethylenic bond and/or the substituent on the quinoline ring is a methyl or ethoxy group or a chlorine atom.

Of the attached drawings, Figs 1 to 4 show the infrared absorption spectra of the chromogenic compounds respectively produced in Examples 1, 2, 3 and 6.

Chromogenic compounds of the present invention have the following features. They have a lower melting point than that of the compound disclosed in GB-A 2 136 823 and are readily soluble in a cheap solvent among solvents used for pressure- or heat sensitive recording paper, if the hydrocarbon residue (R) in the formula (I) has 6 or more carbon atoms. In addition, they are more stable to light when in the form of solution. They produce a color image of deep hue, if one of the two –OR groups on the styryl group is at the ortho position with respect to the ethylenic bond.

(Incidentally, a styryl quinoline compound in the form of solution gradually loses its color forming ability in proportion to the amount of light to which it is exposed, because the double bond of the styryl group contained therein undergoes chemical reactions (presumably dimerization) upon exposure to light). With the chromogenic compound of the present invention, this disadvantage is eliminated if one –OR group occupies the ortho position with respect to the ethylenic bond. Further, the chromogenic compounds of the present invention are readily desensitized by a desensitizing ink.

The present invention also provides a process for the preparation of a chromogenic compound of formula (I), which process comprises condensing a quinaldine derivative of formula (II) wherein the quinoline ring is unsubstituted or substituted in the 6-position by a $C_1$–$C_4$ alkoxy or a $C_1$–$C_4$ alkyl group or a halogen atom, with a substituted benzaldehyde of formula (III) wherein R is as defined above. The condensation is promoted by the presence of a dehydrating agent.

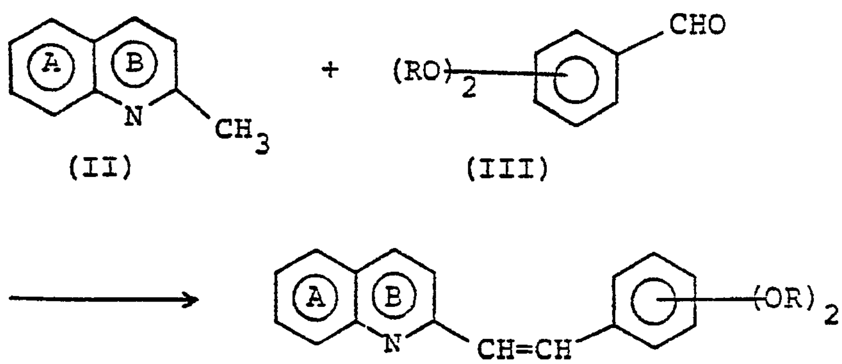

(II) (III)

(I)

Alternatively, the present invention provides a further process for the preparation of a chromogenic compound of formula (I), which process comprises condensing a quinaldine derivative of formula (II) with a dihydroxybenzaldehyde of formula (IV) and alkylating the dihydroxystyrylquinoline of formula (V) thus obtained, for example with an alkyl halide, so as to convert the two hydroxy groups into groups OR wherein R is as defined above.

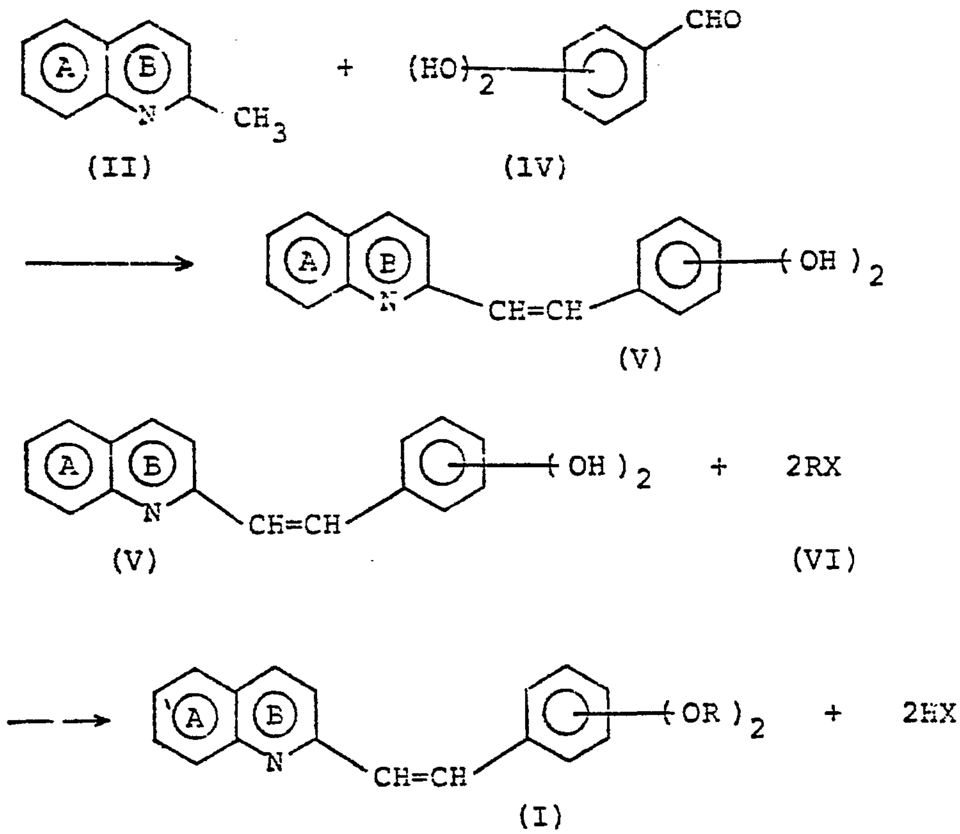

(II) (IV) (V) (V) (VI) (I)

As compared with the first process, the second one is faster in condensation reaction and higher in yields.

A chromogenic compound of the present invention is almost colorless but it rapidly changes into yellow upon contact with an electron acceptor. Therefore, it is useful as a color developing agent for pressure- or heat-sensitive recording paper (copying paper).

A chromogenic compound of the present invention is superior to conventional yellow color developing agents in the following points.

1) It produces an image of higher color density, and it is stabler to light.
2) It is more soluble in organic solvents.
3) It is less liable to spontaneous color formation in case of coating the solution thereof with a paper.
4) It is less liable to loss by sublimation.
5) It is more easily desensitized with an antisensitive ink.

s Table 1 shows, a chromogenic compound of the present invention is superior in color density of the image, light-fastness of the image, and desensitization to the comparative compounds disclosed in GB-A 2 136 823.

Table 1

| | | | Color density | de-sensiti-zation |
|---|---|---|---|---|
| The present invention | Compound No.1 | 1-(2,4-dioctyloxy-phenyl)-2-(2'-quinolyl)ethylene | 0.96 | 0.23 |
| | Compound No.3 | 1-(2,4-didodecyloxy-phenyl)-2-(2'-quinolyl)ethylene | 0.79 | 0.16 |
| GB-A-2136823 | Compound No. 13 | 1-(3-methoxy-4-octyloxyphenyl)-2-(2'-quinolyl)-ethylene | 0.77 | 0.56 |
| | Compound No. 14 | 1-(3-methoxy-4-dodecyloxyphenyl)-2-(2'-quinolyl)-ethylene | 0.72 | 0.48 |

The present invention:

Compound No. 1:

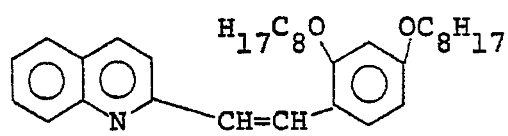

Compound No. 3:

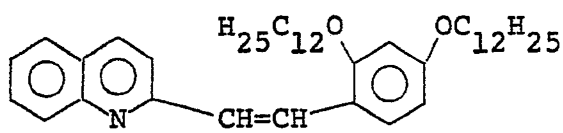

GB-A 2 136 823:

Compound No. 13:

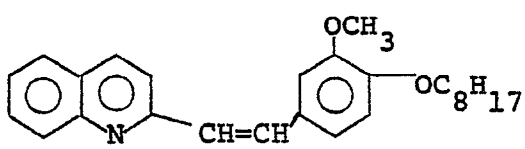

Compound No. 14:

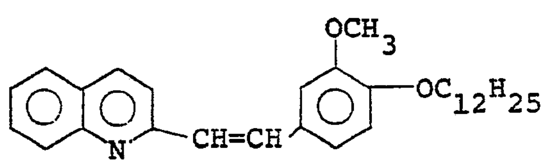

The color density and desensitization are measured by the following methods.

(I) Color density

A sheet of base paper is coated by doctor knife with a 3% solution of sample compound in KMC (alkylnaphthalene solvent for pressure-sensitive recording paper, made by Kureha Chemical Industry Co., Ltd., Japan) at a coating weight of 5 g/m². With the coated paper placed on a color-developer sheet (clay-coated paper), pressure is applied by rolling to effect color formation. Thirty seconds after color formation, the color density is determined according to the amount of light absorbed by the color which is measured by using an integrating type spectrophotometer (Model UV-200, made by Shimadzu Seisakusho Ltd., Japan).
(2) Desensitization

At first, coated-front sheet (clay-coated paper) is coated with a desensitizing ink composed mainly of polypropylene glycol having an average molecular weight of about 3000 at a coating weight of 2 to 3 g/m². On the other hand, coated-back sheet is prepared by coating with microcapsules containing 3 parts by weight of the chromogenic compound and 97 parts by weight of KMC (alkylnaphthalene solvent for pressure-sensitive recording paper, made by Kureha Chemical Industry Co., Ltd.) according to the method described in Example 9. With the coated-back sheet placed on the coated-front sheet, the microcapsules are broken by pressure rolling. Three days later, the color density on the coated-front sheet is determined in terms of Macbeth reflection density. For reference, the same procedure as above is repeated except that no desensitizing ink is used. Desensitization is expressed in relative value and the recording paper having the relative value of not more than 0.25 is used in practical.

A chromogenic compound of the present invention can be encapsulated into microcapsules for pressure-sensitive recording paper by any known process. For example, microencapsulation by coacervation as disclosed in US-A 2 806 457 and US-A 2 800 458 may be adopted. The other suitable process is disclosed in JP-B 4 514 039 (1970).

The microcapsules containing a chromogenic compound of the present invention may be coated onto either a supporting paper or a paper coated with electron acceptor. In the former case two pieces of paper, one coated with microcapsules and the other coated with an electron acceptor, are required for color formation, and in the latter case the color formation is accomplished on a single piece of paper.

Upon application of pressure in use of the pressure-sensitive recording paper, the microcapsules are broken and the chromogenic compound contained therein comes into contact with the adjacent electron acceptor to effect color formation. The electron acceptor may be on the same paper as for the microcapsules or on the paper separate from the one coated with the microcapsules.

As an electron acceptor, organic acids, acid clay, activated clay, phenol-formaldehyde resin, metal salts of aromatic carboxylic acids, and aromatic hydroxy compounds such as bisphenol A may be exemplified.

As a wall material of microcapsules, polyvinyl alcohol, polyacrylic acid, hydroxyethyl cellulose, methyl cellulose, carboxymethyl cellulose, polyacrylamide, polyvinyl pyrrolidone, gelatin, and starch may be exemplified.

Preferable chromogenic compounds of the present invention are 1-(2,4-dioctyloxyphenyl)-2-(2′-quinolyl)ethylene,
1-(2,4-dibenzyloxyphenyl)-2-(2′-quinolyl)ethylene,
1-(2,4-didodecyloxyphenyl)-2-(2′-quinolyl)ethylene,
1-(4,5-didodecyloxyphenyl)-2-(6′-ethoxy-2′-quinolyl)ethylene,
1-(4,5-dibenzyloxyphenyl)-2-(6′-ethoxy-2′-quinolyl)ethylene,
1-(5,6-dioctyloxyphenyl)-2-(2′-quinolyl)ethylene
1-(2,4-dioctyloxyphenyl)-2-(6′-chloro-2′-quinolyl)ethylene, and
1-(2,4-dioctyloxyphenyl)-2-(6′-methyl-2′-quinolyl)ethylene.
Most suitable among them is
1-(2,4-dioctyloxyphenyl)-2-(2′-quinolyl)ethylene.

The chromogenic compound of the present invention is highly soluble, stable to light, capable of forming color images of deep hue, and readily desensitized.

The present invention will be explained in more detail with reference to the following non-limitative examples.

EXAMPLE I

Synthesis of I-(2,4-dioctoxyphenyl)-2-(2′-quinolyl)ethylene (Compound No. I)

I4.3 g of quinaldine, I3.8 g of 2,4-dihydroxybenzaldehyde, and 30 ml of acetic anhydride were reacted under reflux for 2 hours. The reaction product was added to 200 ml of water. After stirring for a while, the resulting precipitates were filtered out, washed, and dried, and then 24 g of slightly yellowish crystals having a melting point of I26 to I33.5°C was obtained. This compound was identified as I-(2,4-diacetoxyphenyl)-2-(2′-quinolyl)ethylene of the following structural formula.

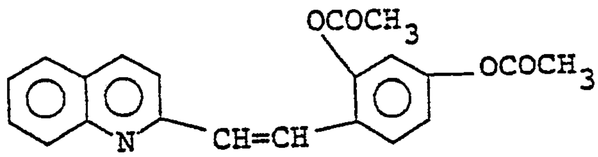

34.7 g of this compound was reacted with 48.2 g of n-octyl bromide and 33.l g of anhydrous potassium carbonate in 60 ml of diethylene glycol monomethyl ether at l40°C for 2 hours. After cooling, the reaction product was filtered out and washed. The pastelike reaction product was dispersed into 200 ml of water. The dispersed particles were filtered out, washed, and dried, and then 40 g of grayish white crystals having a melting point of 5l to 54.3°C were obtained. This compound was identified as l-(2,4-dioctoxyphenyl)-2-(2′-quinolyl)ethylene of the following structural formula.

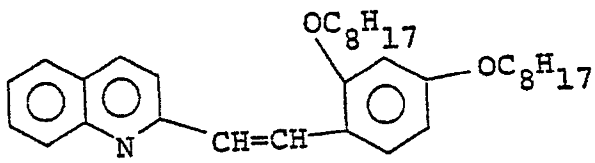

When dissolved in 95 % acetic acid, this compound gave the $\lambda_{max}$at 433 nm. A toluene solution of this compound was colorless, but it formed a color giving the $\lambda_{max}$ at 455 nm on the clay-coating paper of pressure-sensitive recording paper. This color was nearly orange.

The results of elemental analysis were as follows:

| | C (%) | H (%) | N (%) |
|---|---|---|---|
| calcd. for $C_{33}H_{45}NO_2$ | 81.31 | 9.24 | 2.87 |
| Found | 81.44 | 9.39 | 2.85 |

The infrared spectrum of Compound No. l is shown in Fig. l.

EXAMPLES 2 to 8

The same procedure as in Example l was repeated except that quinaldines, dihydroxybenzaldehydes, and halogenated hydrocarbons as shown in Table 2 were used, and chromogenic compounds as shown in Table 2 were obtained.

All of them were colorless in toluene solution but formed a yellow color upon reaction with an eletron acceptor. The chromogenic compounds obtained in Examples 2 and 3 remarkably formed a color image of deep hue on the clay coating paper.

Table 2

| Compound | Quinaldines | dihydroxy-benzaldehydes | halogenated hydrocarbons | Chromogenic Compounds | Property | Melting point (°C) | I.R. |
|---|---|---|---|---|---|---|---|
| 2 | 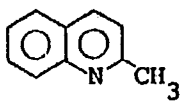 | 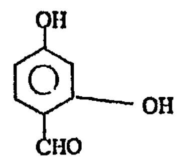 | 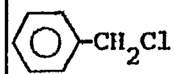 | 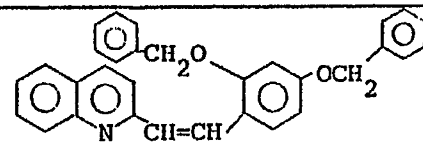 | Yellow color 424 nm (AcOH) 450 nm (clay) | 120 ~ 126.5 | Fig. 2 |
| 3 | 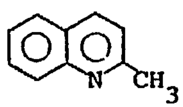 | 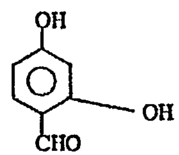 | $C_{12}H_{25}Br$ | 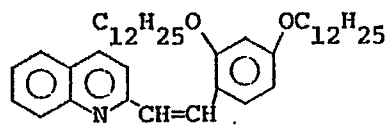 | Yellow color 433 nm (AcOH) 455 nm (clay) | 50.5 ~ 52.7 | Fig. 3 |
| 4 | 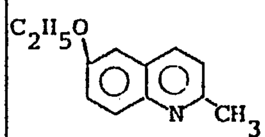 | 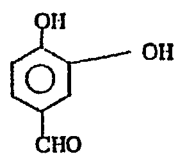 | $C_{12}H_{25}Br$ | 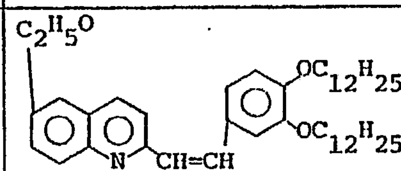 | Yellow color 423 nm (AcOH) | 72 ~ 78 | - |
| 5 | 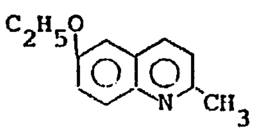 | 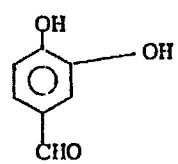 | 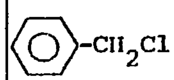 | 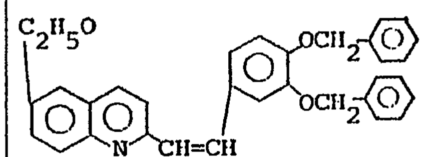 | Yellow color 420 nm (AcOH) | 125 ~ 132 | - |

Table 2 (cont'd)

| Compound | Quinaldines | dihydroxy-benzaldehydes | halogenated hydrocarbons | Chromogenic Compounds | Property | Melting point (°C) | I.R. |
|---|---|---|---|---|---|---|---|
| 6 | 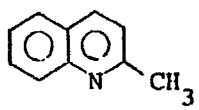 | 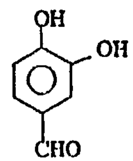 | $C_8H_{17}Br$ | 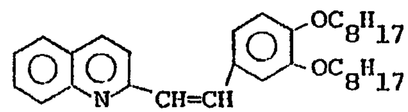 | Yellow color 426 nm (AcOH) | 45 ~ 53 | Fig. 4 |
| 7 | 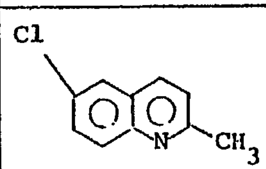 | 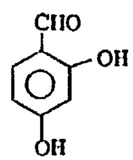 | $C_8H_{17}Br$ | 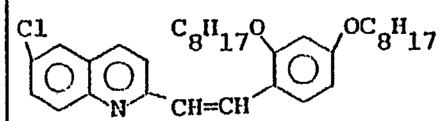 | Yellow color 435 nm (AcOH) 455 nm (clay) | 81 ~ 86 | - |
| 8 | 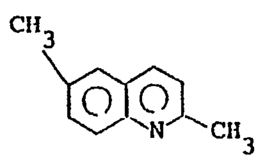 | 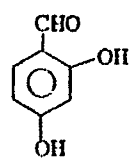 | $C_8H_{17}Br$ | 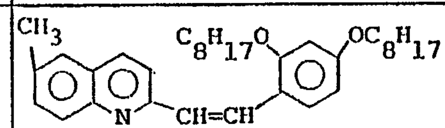 | Yellow color 432 nm (AcOH) 451 nm (clay) | 55.5 ~ 61 | - |

| Elemental analysis of Compound No. 2 | C (%) | H (%) | N (%) |
|---|---|---|---|
| calcd. for $C_{31}H_{25}NO_2$ | 83.97 | 5.64 | 3.16 |
| Found | 83.73 | 5.64 | 2.91 |

| Elemental analysis of Compound No. 3 | C (%) | H (%) | N (%) |
|---|---|---|---|
| calcd. for $C_{41}H_{61}NO_2$ | 82.13 | 10.18 | 2.33 |
| Found | 81.87 | 10.33 | 2.24 |

| Elemental analysis of Compound No. 4 | C (%) | H (%) | N (%) |
|---|---|---|---|
| calcd. for $C_{43}H_{65}NO_3$ | 80.24 | 10.10 | 2.17 |
| Found | 80.83 | 9.95 | 2.23 |

| Elemental analysis of Compound No. 5 | C (%) | H (%) | N (%) |
|---|---|---|---|
| calcd. for $C_{33}H_{29}NO_3$ | 81.31 | 5.95 | 2.87 |
| Found | 81.05 | 5.46 | 2.77 |

| Elemental analysis of Compound No. 6 | C (%) | H (%) | N (%) |
|---|---|---|---|
| calcd. for $C_{33}H_{45}NO_2$ | 81.31 | 9.24 | 2.87 |
| Found | 80.32 | 9.66 | 2.44 |

| Elemental analysis of Compound No. 7 | C (%) | H (%) | N (%) |
|---|---|---|---|
| calcd. for $C_{33}H_{44}NO_2Cl$ | 75.93 | 8.43 | 2.68 |
| Found | 75.82 | 8.33 | 2.76 |

| Elemental analysis of Compound No. 8 | C (%) | H (%) | N (%) |
|---|---|---|---|
| calcd. for $C_{34}H_{47}NO_2$ | 81.43 | 9.38 | 2.79 |
| Found | 80.98 | 9.42 | 2.83 |

Example 9

Preparation of pressure-sensitive recording paper.

The chromogenic compounds obtained in Examples 1 to 8 were used. For comparison, 2-(4-octyloxystyryl)quinoline (disclosed in GB-A 2 136 823) and
2-(3-methoxy-4-dodecyloxystyryl)quinoline were used.

At first, the microencapsulation of the chromogenic compound was performed as follows:

Six parts by weight of the chromogenic compound was dissolved in 94 parts by weight of isopropylbiphenyl. Separately, 24 parts of gelatin and 24 parts of gum arabic were dissolved in 400 parts of water, and the resulting solution was adjusted to pH 7. The first solution was added to the second solution, and the mixture was emulsified with homogenizer. To this emulsion was added 1000 parts of hot water, followed by stirring at 50°C for 30 minutes. One part of 10% aqueous solution of sodium hydroxide was added, followed by stirring at 50°C for 30 minutes. Dilute acetic acid was slowly added to adjust the pH thereof to 4.5. Stirring was continued at 50°C for about 1 hour. The emulsion was cooled to 0 to 5°C and stirred for 30 minutes. 35 parts of 4% aqueous solution of glutaraldehyde was slowly added thereto thereby forming microcapsules. A dilute aqueous solution of sodium hydroxide was added to adjust the pH to 6. Stirring was continued at room temperature for several hours to complete microencapsulation. The microcapsule dispersion thus prepared was applied to paper, followed by drying, to obtain microcapsules coating paper (pressure-sensitive upper paper).

Each of the upper paper samples was placed on clay coating paper (pressure-sensitive lower paper), with the microcapsules and the coated clay in contact with each other. Pressure was applied to the pressure-sensitive paper by rolling to effect color formation. The color density on the lower paper was measured with a reflection densitometer. The results are shown in Table 3. To evaluate the stability to light of the chromogenic compounds, the same procedure as above was repeated after the upper paper had been exposed to sunlight for 20 minutes or 40 minutes. The results are also shown in Table 3. The data are compared in percentage in parentheses. Also, the desensitization of the chromogenic compounds were measured by the aforementioned method. The results are also shown in Table 3.

As seen from Table 3, the chromogenic compounds of the present invention are comparable with that disclosed in GB-A 2 136 823 and are superior in desensitization to that disclosed in GB-A 2 136 823. The stability to light is greatly improved in the case of chromogenic compounds in which one of the substituent groups on the styryl group is at the ortho position, as in Examples 1, 2, 3, 7 and 8.

Table 3

| Chromogenic Compound | Color Density (Exposing Time) | | | Desensitization |
|---|---|---|---|---|
| | 0 min. | 20 min. | 40 min. | |
| 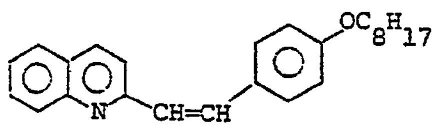 (Comparative Example 1) | 0.673 (100) | 0.461 (68) | 0.433 (64) | - |
| 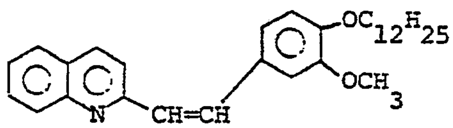 (Comparative Example 2) | 0.747 (100) | 0.495 (66) | 0.459 (61) | 0.48 |
| 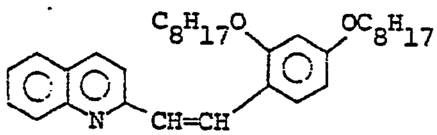 (Example 1) | 0.858 (100) | 0.749 (87) | 0.718 (84) | 0.23 |
| 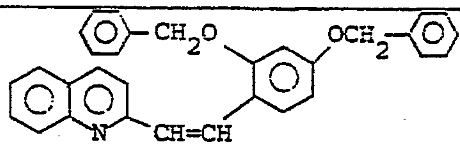 (Example 2) | 0.812 (100) | 0.690 (85) | 0.657 (81) | - |
| 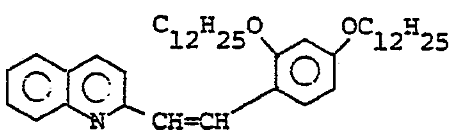 (Example 3) | 0.850 (100) | 0.756 (89) | 0.705 (83) | 0.16 |
| 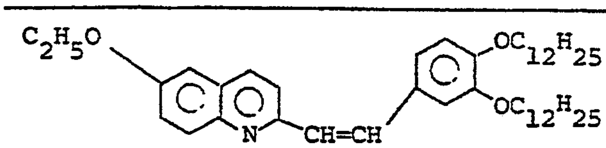 (Example 4) | 0.759 (100) | 0.523 (69) | 0.493 (65) | 0.25 |
| 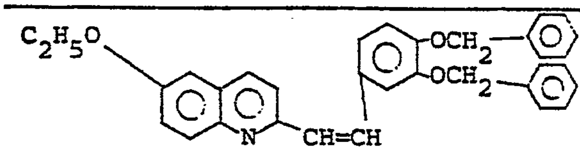 (Example 5) | 0.734 (100) | 0.521 (71) | 0.491 (67) | - |

Table 3 (Cont'd)

| Chromogenic Compound | Color Density (Exposing Time) | | | Desensitization |
|---|---|---|---|---|
| | 0 min. | 20 min. | 40 min. | |
| 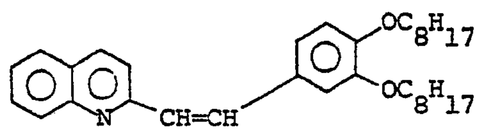 (Example 6) | 0.767 (100) | 0.522 (68) | 0.483 (63) | 0.25 |
| 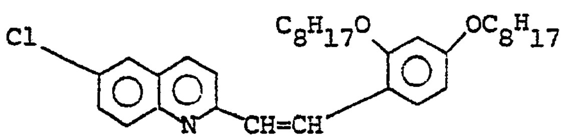 (Example 7) | 0.811 (100) | 0.673 (83) | 0.640 (79) | 0.24 |
| 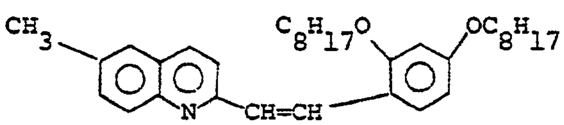 (Example 8) | 0.831 (100) | 0.673 (81) | 0.631 (76) | 0.23 |

EXAMPLE 10

Preparation of heat-sensitive recording paper

30 parts by weight of l-(2,4-benzyloxyphenyl)-2-(2′-quinolyl)ethylene (obtained in Example 2) was crushed in l50 parts by weight of l0 % aqueous solution of polyvinyl alcohol and 65 parts by weight of water for l hour using a ball mill until the particle size was l to 3 μm. (The product was designated as component A.) Separately, 35 parts by weight of bisphenol A was crushed in l50 parts by weight of l0 % aqueous solution of polyvinyl alcohol and 65 parts by weight of water using a ball mill until the particle size was l to 3 μm. (The product was designated as component B.) 3 parts by weight of component A and 67 parts by weight of component B were mixed and the resulting mixture was applied to base paper at a coating weight of about 5 g/m². The resulting heat-sensitive paper rapidly formed a yellow color upon heating with a thermal pen. The color image was stable to light and resistant to moisture.

## Claims

1. A chromogenic compound represented by the formula (I):

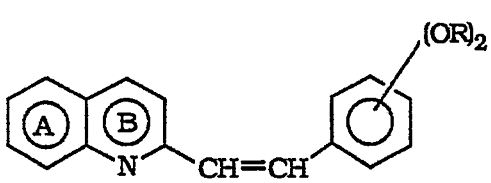

(I)

wherein R represents a $C_6$–$C_{12}$ alkyl group or a benzyl group and the quinoline ring is unsubstituted or substituted in the 6-position by a $C_1$–$C_4$ alkoxy or a $C_1$–$C_4$ alkyl group or a halogen atom.

2. A chromogenic compound according to claim 1, wherein R represents an alkyl group of 8 to 12 carbon atoms.

3. A chromogenic compound according to claim 1 or 2, wherein one of the OR substituents is in the orthoposition with respect to the ethylenic bond.

4. A chromogenic compound according to any one of claims 1 to 3, wherein the substituent on the quinoline ring is a methyl or ethoxy group or a chlorine atom.

5. A chromogenic compound according to claim 1, which is 1-(2,4-dioctyloxyphenyl)-2-(2′-quinolyl)ethylene,
1-(2,4-dibenzyloxyphenyl-2-(2′-quinolyl)ethylene,
1-(2,4-didodecyloxyphenyl)-2-(2′-quinolyl)ethylene,
1-(4,5-didodecyloxyphenyl)-2-(6′-ethoxy-2′-quinolyl)-ethylene, 1-(4,5-dibenzyloxyphenyl)-2-(6′ethoxy-2′-quinolyl)ethylene, 1-(5,6-dioctyloxyphenyl)-2-(2′-quinolyl)-ethylene, 1-(2,4-dioctyloxyphenyl)-2-(6′-chloro-2′-quinolyl)ethylene, or 1-(2,4-dioctyloxyphenyl)-2-(6′-methyl-2′-quinolyl)ethylene.

6. A chromogenic compound according to claim 5, which is 1-(2,4-dioctyloxyphenyl)-2-(2′-quinolyl)ethylene.

7. A process for the preparation of a chromogenic compound of formula (I) as defined in claim 1, which process comprises condensing a quinaldine derivative of formula (II):

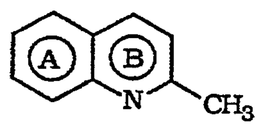

(II)

wherein the quinoline ring is unsubstituted or substituted in the 6-position by a $C_1$–$C_4$ alkoxy or a $C_1$–$C_4$ alkyl group or a halogen atom, with a substituted benzaldehyde of formula (III):

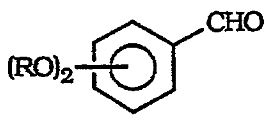

(III)

wherein R is as defined in claim 1.

8. A process for the preparation of a chromogenic compound of formula (I) as defined in claim 1, which process comprises condensing a quinaldine derivative of formula (II) as defined in claim 7 with a dihydroxybenzaldehyde of formula(IV):

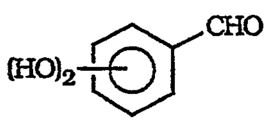

(IV)

and alkylating the dihydroxystyrylquinoline of formula (V) thus obtained:

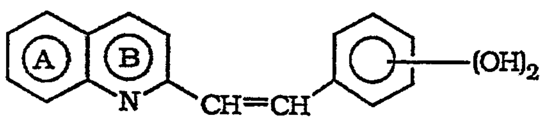

(V)

so as to convert the two hydroxy groups into groups OR wherein R is as defined in claim 1.

9. Microcapsules suitable for use in the preparation of a pressure- or heat -sensitive recording paper, in which microcapsules there is encapsulated a solution of at least one chromogenic compound of formula (I) as claimed in any one of claims 1 to 6 in an organic solvent therefor.

10. A pressure- or heat -sensitive recording paper incorporating at least one chromogenic compound of formula (I) as claimed in any one of claims 1 to 6 as color former.

11. A recording paper according to claim 10, wherein an organic acid, acid clay, activated clay, phenolformaldehyde resin, a metal salt of aromatic carboxylic acid or an aromatic hydroxy compound is present as an electron acceptor.

12. A recording paper according to claim 10 or 11, wherein the at least one chromogenic compound of formula (I) is encapsulated in solution in an organic solvent therefor in microcapsules.

13. Use as a color former for pressure- or heat-sensitive recording papers of at least one chromogenic compound of formula (I) as claimed in any one of claims 1 to 6.

## Patentansprüche

1. Chromogene Verbindung der Formel (I)

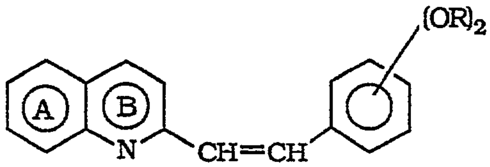

(I)

worin R eine $C_6$–$C_{12}$-Alkylgruppe oder eine Benzylgruppe bedeutet und der Chinolinring unsubstituiert oder in der 6-Stellung durch eine $C_1$–$C_4$-Alkoxygruppe oder eine $C_1$–$C_4$-Alkylgruppe oder ein Halogenatom substituiert ist.

2. Chromogene Verbindung nach Anspruch 1, worin R eine Alkylgruppe mit 8 bis 12 Kohlenstoffatomen darstellt.

3. Chromogene Verbindung nach Anspruch 1 oder 2, worin einer der Substituenten OR in bezug auf die ethylenische Bindung in der ortho-Stellung steht.

4. Chromogene Verbindung nach einem der Ansprüche 1 bis 3, worin der Substituent am Chinolinring eine Methylgruppe, eine Ethoxygruppe oder ein Chloratom ist.

5. Chromogene Verbindung nach Anspruch 1, nämlich 1-(2,4-Dioctyloxyphenyl)-2-(2′-chinolyl)-ethylen, 1-(2,4-Dibenzyloxyphenyl-2-(2′-chinolyl)-ethylen, 1-(2,4-Didodecyloxyphenyl)-2-(2′-chinolyl)-ethylen, 1-(4,5-Didodecyloxyphenyl)-2-(6′-ethoxy-2′-chinolyl)-ethylen, 1-(4,5-Dibenzyloxyphenyl)-2-(6′-ethoxy-2′-chinolyl)-ethylen, 1-(5,6-Dioctyloxyphenyl)-2-(2′-chinolyl)-ethylen, 1-(2,4-Dioctyloxyphenyl)-2-(6′-chlor-2′-chinolyl)-ethylen oder 1-(2,4-Dioctyloxyphenyl)-2-(6′-methyl-2′-chinolyl)-ethylen.

6. Chromogene Verbindung nach Anspruch 5, nämlich 1-(2,4-Dioctyloxyphenyl)-2-(2′-chinolyl)-ethylen.

7. Verfahren zur Herstellung einer chromogenen Verbindung der Formel (I), wie sie in Anspruch 1 definiert ist, welches darin besteht, ein Chinaldinderivat der Formel (II)

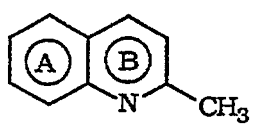

(II)

dessen Chinolinring unsubstituiert oder in der 6-Stellung durch eine $C_1$–$C_4$-Alkoxygruppe, eine $C_1$–$C_4$-Alkylgruppe oder ein Halogenatom substituiert ist, mit einem substituierten Benzaldehyd der Formel (III)

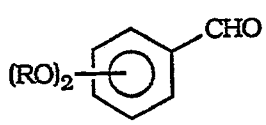

(III)

worin R die in Anspruch 1 angegebene Definition besitzt, zu kondensieren.

8. Verfahren zur Herstellung einer chromogenen Verbindung der Formel (I), wie sie in Anspruch 1 definiert ist, welches darin besteht, ein Chinaldinderivat der in Anspruch 7 definierten Formel (II) mit einem Dihydroxybenzaldehyd der Formel (IV)

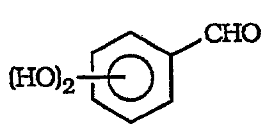

(IV)

zu kondensieren und das in dieser Weise erhaltene Dihydroxystyrylchinolin in der Formel (V)

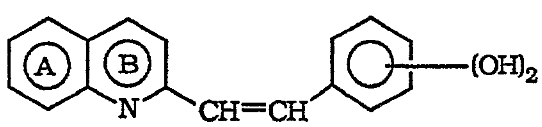

(V)

zu alkylieren, um die beiden Hydroxygruppen in OR-Gruppen umzuwandeln, worin R die in Anspruch 1 angegebenen Bedeutungen besitzt.

9. Mikrokapseln für die Herstellung eines druck- oder wärmeempfindlichen Durchschreibepapiers, welche Mikrokapseln eine Lösung mindestens einer chromogenen Verbindung der Formel (I) gemäß einem der Ansprüche 1 bis 6 in einem dafür geeigneten organischen Lösungsmittel eingekapselt enthalten.

10. Druck- oder wärmeempfindliches Durchschreibepapier enthaltend mindestens eine chromogene Verbindung der Formel (I) gemäß einem der Ansprüche 1 bis 6 als Farbbildner.

11. Durchschreibepapier nach Anspruch 10, worin eine organische Säure, ein saurer Ton, ein aktivierter Ton, ein Phenol-Formaldehyd-Harz, ein Metallsalz einer aromatischen Carbonsäure oder eine aromatische Hydroxyverbindung als Elektronenakzeptor vorhanden ist.

12. Durchschreibepapier nach Anspruch 10 oder 11, worin die mindestens eine chromogene Verbindung der Formel (I) in Form einer Lösung in einem dafür geeigneten organischen Lösungsmittel in Mikrokapseln eingekapselt ist.

13. Verwendung mindestens einer chromogenen Verbindung der Formel (I) gemäß einem der Ansprüche 1 bis 6 als Farbbildner für druck- oder wärmeempfindliche Durchschreibepapiere.

**Revendications**

1. Un composé chromogène représenté par la formule (I):

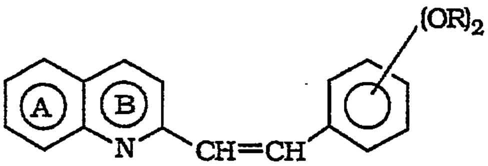

(I)

dans laquelle R représente un groupe alkyle ou un groupe benzyle $C_6$–$C_{12}$, et l'anneau quinoline est non-substitué ou substitué dans la position 6 par un groupe alkoxy $C_1$–$C_4$ ou un groupe alkyle $C_1$–$C_4$ ou un atome halogène.

2. Un composé chromogène selon la revendication 1, dans lequel R représente un groupe alkyle de 8 à 12 atomes de carbone.

3. Un composé chromogène selon la revendication 1, ou 2, dans lequel un des substituants OR est dans la position ortho par rapport à la liaison éthylénique.

4. Un composé chromogène selon une quelconque des revendications 1 à 3, dans lequel le substituant sur l'anneau quinoline est une groupe méthyle ou ethoxy, ou un atome de chlore.

5. Un composé chromogène selon la revendication 1, qui est de l'éthylène 1-(2,4-dioctyloxyphényle)-2-(2′-quinolyle); de l'éthylène 1-(2,4-dibenzyloxyphényle)-2-(2′-quinolyle); de l'éthylène 1-(2,4-didodecyloxyphyényle)-2-(2′quinolyle); de l'éthylène 1-(4,5-didodecyloxyphényle)-2-(6′-ethoxy-2′-quinolyle); de l'éthylène 1-(4,5-dibenzyloxyphenyle)-2-(6′ ethoxy-2′-quinolyle); de l'éthylène 1-(5,6-dioctyloxyphenyle)-2-(2′-quinolyle); de l'éthylène 1-(2,4-dioctyloxyphenyle)-2-(6′-chloro-2′-quinolyle); ou de l'éthylène 1-(2,4-dioctyloxyphenyle)-2-(6′-méthyle-2′quinolyle).

6. Un composé chromogène selon la revendication 5, qui est de l'éthylène 1-(2,4-dicotyloxyphenyle)-2-(2′-quinolyle).

7. Une méthode pour la préparation d'un composé chromogène de la formule (I) selon la définition de la revendication 1, la dite méthode comprenant la condensation d'un dérivé de quinaldine de formule (II):

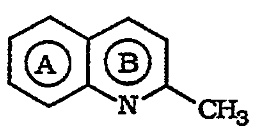

(II)

l'anneau quinoline, dans cette formule étant non substitué ou substitué dans la position 6 par un groupe alkoxy $C_1$–$C_4$ ou un groupe alkyle $C_1$–$C_4$ ou un atome halogène, avec une benzaldéhyde substituée de formule (III):

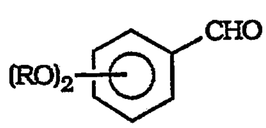

(III)

R étant comme défini dans la revendication I.

8. Une méthode pour la préparation d'un composé chromogène de la formule (I) selon la definition de la revendication 1, la dite méthode comprenant la condensation d'un dérivé de quinaldine de formule (II) selon la définition de la revendication 7, avec une dihydroxybenzaldéhyde de formule (IV):

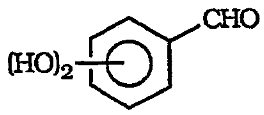

(IV)

et alkylation de la dihydroxystyrylquinoline de formule (V) ainsi obtenue:

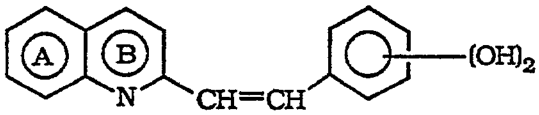

(V)

de façon à convertir les deux groupes hydroxy en des groupes OR dans lesquels R est comme défini dans la revendication 1.

9. Microcapsules appropriées pour utilisation dans la préparation d'un papier enregistreur sensible à la pression ou à la chaleur, et dans lesquelles est encapsulée,

dans un solvant organique approprié, une solution d'au moins un composé chromogène de la formule (I) comme revendiqué dans une quelconque des revendications 1 à 6.

10. Un papier enregistreur sensible à la pression ou sensible à la chaleur, incorporant au minimum un composé chromogène de la formule (I) comme revendiqué dans une quelconque des revendications 1 à 6, ce composé étant incorporé comme formeur de coloration.

11. Un papier enregistreur selon la revendication 10, dans lequel un acide organique, de l'argile acide, de l'argile activée, de la résine phénolformaldéhyde, un sel métallique d'acide carboxylique ou un composé hydroxy-aromatique, est présent en tant qu'accepteur d'électrons.

12. Un papier enregistreur selon la revendication 10 ou 11, dans lequel le dit au moins un composé chromogène de la formule (I) est encapsulé en solution dans un solvant organique approprié en microcapsules.

13. Utilisation comme formeur de coloration pour des papiers enregistreurs sensibles à la pression ou sensibles à la chaleur d'au moins un composé chromogène de la formule (I) comme revendiqué dans une quelconque des revendications 1 à 6.

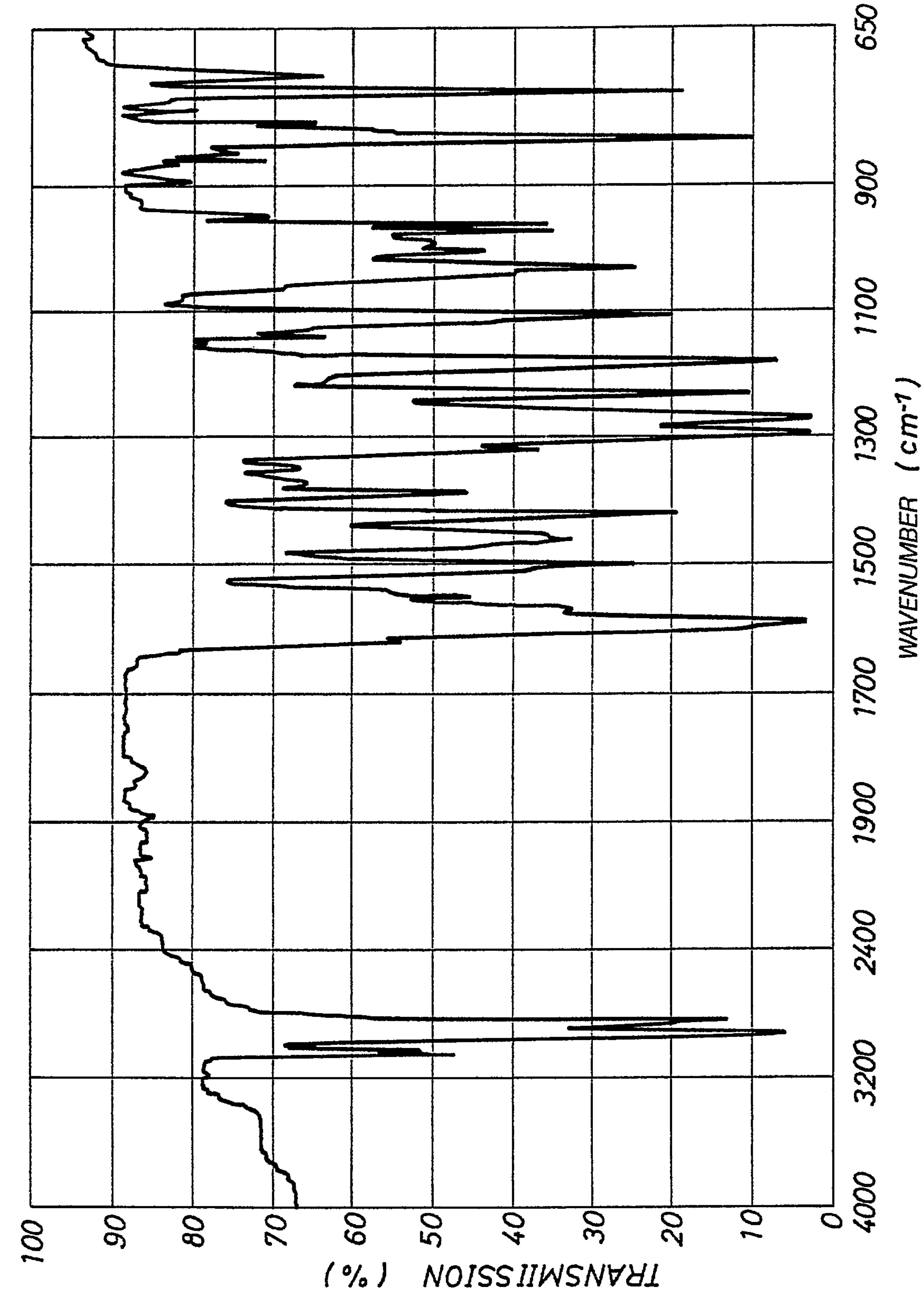

TRANSMISSION (%)
100
90
80
70
60
50
40
30
20
10
0
WAVENUMBER (cm-1)
4000
3200
2400
1900
1700
1500
1300
1100
900
650


Fig.1

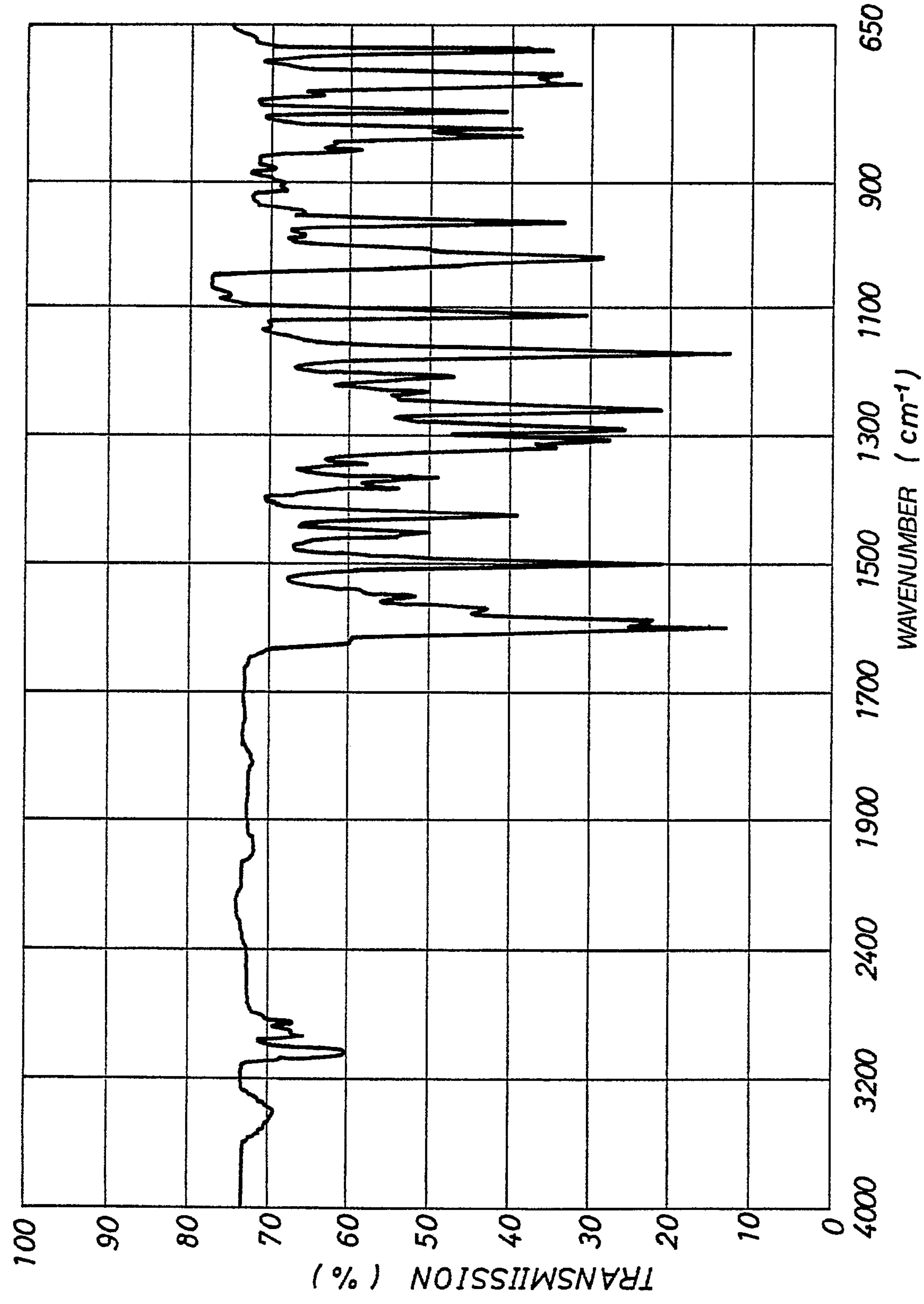

TRANSMISSION ( % )
WAVENUMBER ( cm-1 )
100
90
80
70
60
50
40
30
20
10
0
4000
3200
2400
1900
1700
1500
1300
1100
900
650


Fig.2

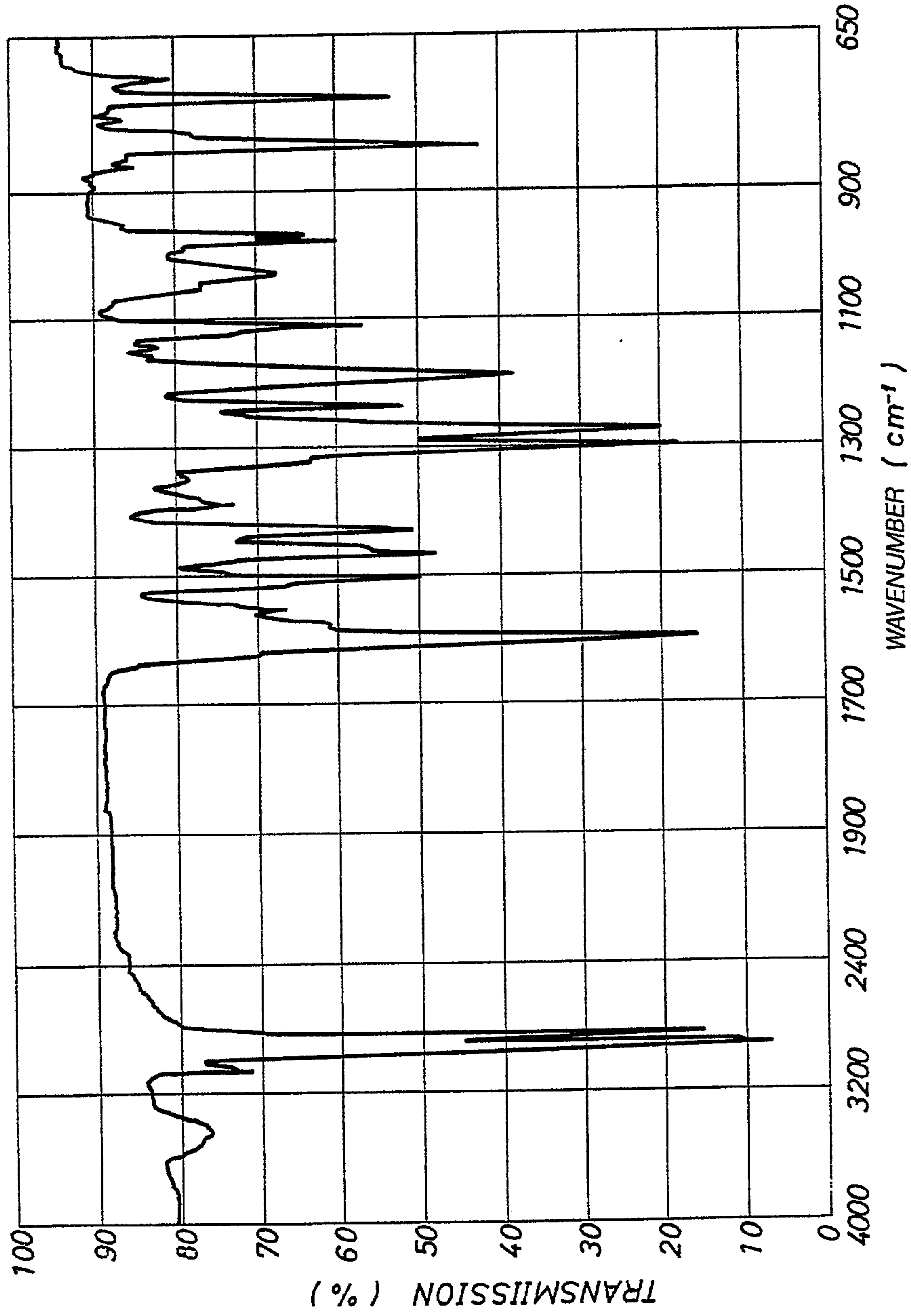

TRANSMISSION ( % )
100
90
80
70
60
50
40
30
20
10
0
WAVENUMBER ( cm-1 )
4000
3200
2400
1900
1700
1500
1300
1100
900
650


Fig. 3

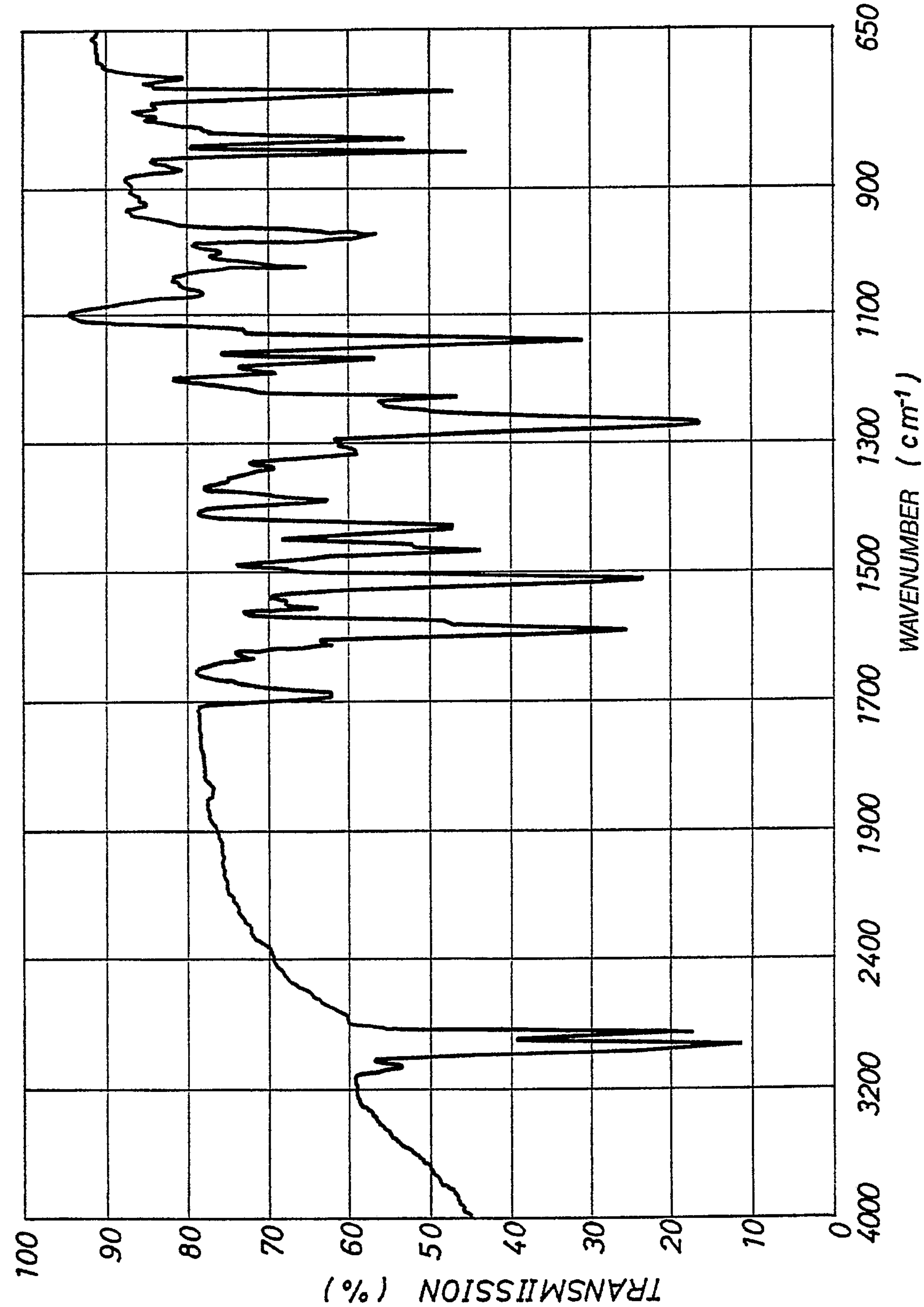
100
90
80
70
60
50
40
30
20
10
0
TRANSMIISSION (%)
4000
3200
2400
1900
1700
1500
1300
1100
900
650
WAVENUMBER (cm-1)

Fig. 4